(19) Europäisches Patentamt
European Patent Office
Office européen des brevets

(11) **EP 2 525 210 B1**

(12) **EUROPÄISCHE PATENTSCHRIFT**

(45) Veröffentlichungstag und Bekanntmachung des Hinweises auf die Patenterteilung:
**04.06.2014 Patentblatt 2014/23**

(51) Int Cl.:
*G01N 21/05* (2006.01)    *G01N 21/31* (2006.01)
*G01N 21/33* (2006.01)    *G01N 33/00* (2006.01)

(21) Anmeldenummer: **11166178.1**

(22) Anmeldetag: **16.05.2011**

(54) **Vorrichtung und Verfahren zur Bestimmung einer Konzentration einer Messkomponente in einem Gas**

Device and method for determining a concentration of a measurement component in a gas

Dispositif et procédé de détermination d'une concentration d'un composant de mesure dans un gaz

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.11.2012 Patentblatt 2012/47**

(73) Patentinhaber: **SICK AG**
**79183 Waldkirch (DE)**

(72) Erfinder:
- **Zöchbauer, Dr., Michael**
  **22393, Hamburg (DE)**
- **Rogge, Dr., Carsten**
  **22844, Norderstedt (DE)**
- **Huttner, Dr., Dominikus**
  **82399, Raisting (DE)**

(74) Vertreter: **Ludewigt, Christoph**
**Sick AG**
**Intellectual Property**
**Erwin-Sick-Strasse 1**
**79183 Waldkirch (DE)**

(56) Entgegenhaltungen:
**DE-A1- 10 242 355     US-A- 3 173 016**
**US-A- 3 925 666       US-A- 4 089 809**
**US-A1- 2001 005 981**

- **"DEFOR Extractive UV Gas Analyzer", , 1. April 2009 (2009-04-01), Seiten 1-4, XP55008905, Germany Gefunden im Internet: URL:http: //www.baytec-inc.com/pdf/defor-ex tractive-analyzer.pdf [gefunden am 2011-10-06]**
- **Frank Imbrogno ET AL: "A comparison of different analytical approaches to the on-line measurement of hydrogen sulfide", The Annual American Gas Association Conference; Montreal, 1. Mai 1996 (1996-05-01), Seiten 1-19, XP55009021, Gefunden im Internet: URL:http: //www.haritec.com/papers/online H2S.pdf [gefunden am 2011-10-07]**

**Beschreibung**

Die Erfindung betrifft eine Vorrichtung zur Bestimmung einer Konzentration einer Messkomponente, nämlich $H_2S$, in einem Gas gemäß dem Oberbegriff des Anspruchs 1.

Für die selektive $H_2S$-Messung werden traditionell Paper-Tape-Verfahren eingesetzt, die eine selektive chemische Reaktion zwischen $H_2S$ und zum Beispiel Bleiacetat verwenden. Nachteilig daran ist, dass Paper-Tape-Verfahren nur quasi kontinuierlich arbeiten, sehr aufwändig sind und insbesondere wartungsintensiv. Eine mit Bleiacetat getränkte Papierrolle hält nur durchschnittlich zwei bis vier Wochen.

Weiterhin sind UV-Simultanspektrometer bekannt, mit denen eine höhere Selektivität durch chemometrische Methoden erreicht werden kann. Simultanspektrometer sind aber technisch sehr aufwändig. Weiter nachteilig ist, dass nur eine begrenzte Selektivität für $H_2S$ erreichbar ist, wenn die Gasmatrix weitere Schwefelkomponenten enthält, insbesondere Mercaptane. Das $H_2S$-Spektrum unterscheidet sich nur geringfügig von den Mercaptanspektren. Schließlich gibt es einfache UV-Fotometer, die nach dem Interferenzenfilter-Korrelationsverfahren arbeiten. Ein solches ist von der Anmelderin unter der Marke DEFOR bekannt. Diese haben aber den Nachteil, dass sie bei der H2S-Messung sehr hohe Querempfindlichkeiten gegenüber anderen Schwefelverbindungen, z.B. $SO_2$, COS, $CS_2$ und insbesondere Mercaptanen, aufweisen. Diese lassen sich durch spektroskopische Mittel oder durch Querempfindlichkeitskorrekturen kaum reduzieren.

Aus der US 3,173,016 ist eine Vorrichtung zur Messung des Quecksilbergehalts in Luft bekannt.

Aus der DE 102 42 355 ist eine Vorrichtung zur Messung von $H_2S$ bekannt.

Ausgehend von diesem Stand der Technik ist es Aufgabe der Erfindung eine alternative Vorrichtung bereitzustellen.

Diese Aufgabe wird gelöst durch eine Vorrichtung mit den Merkmalen des Anspruchs 1.

Die erfindungsgemäße Vorrichtung zur Bestimmung einer Konzentration einer Messkomponente, nämlich $H_2S$, in einem Gas umfasst

- eine Lichtquelle,
- eine Wellenlängenselektionseinheit,
- eine Messküvette,
- eine im optischen Strahlengang parallel dazu angeordnete Referenzküvette,
- wenigstens einen Lichtempfänger
- eine Auswerteeinheit, die aus den Signalen des Lichtempfängers die Konzentration bestimmt wobei das zu untersuchende Gas einerseits der Messküvette und andererseits über eine Absorptionsvorrichtung, die eine die Messkomponente vollständig absorbierende Substanz enthält, der Referenzküvette zugeführt ist.

Wie bei der aus dem Dokument DE 102 42 355 bekannten Vorrichtung wird jetzt als Referenz nicht ein neutrales Gas genommen, sondern das zu untersuchende Gas selbst, allerdings vermindert um die Messkomponente. Dabei fließt das Messgas zunächst unverändert durch die Messküvette. Der Lichtempfänger, der das Messlicht empfängt, "sieht" somit die Summe aus dem eigentlichen Messeffekt (also die Absorption durch die Messkomponente, d.H. $H_2S$) und alle Querempfindlichkeiten. Im Unterschied zur aus dem Dokument DE 102 42 355 bekannten Vorrichtung ist eine separate Referenzküvette vorhanden. In der Zuführung zur Referenzküvette wird die Absorptionsvorrichtung eingefügt, die nun selektiv die Messkomponente ($H_2S$) entfernt, aber die übrigen Komponenten unverändert hindurchlässt. Das so um die Messkomponente verminderte Gas fließt dann durch die Referenzküvette. Der Lichtempfänger, der das Referenzlicht empfängt, "sieht" dann nun nur noch die Querempfindlichkeiten ohne den eigentlichen Messeffekt. Die Konzentration der Messkomponente ergibt sich aus der Differenz von Messlicht zu Referenzlicht.

Der besondere Vorteil liegt einerseits in der Verwendung eines einfachen Fotometers und der andererseits sehr hohen Selektivität, die letztendlich mit dem Absorber erreicht wird. Insgesamt ist damit eine kostengünstige Möglichkeit geschaffen, hochselektiv den Gasgehalt von $H_2S$ zu bestimmen.

Prinzipiell kann die Messkomponente mit einer einfachen Differenzbildung, wie oben erwähnt, bestimmt werden. Um aber eine hohe Langzeitstabilität zu erreichen und driftfrei messen zu können, ist in Weiterbildung der Erfindung vorgesehen, dass sowohl für das die Messküvette durchlaufende Licht als auch für das die Referenzküvette durchlaufende Licht jeweils ein Lichtempfänger vorgesehen ist und bei zwei verschiedenen Wellenlängen - einer, bei der die Messkomponente absorbiert, und einer, bei der sie nicht absorbiert - Messungen vorzunehmen und dann in der Auswerteeinheit mittels der doppelten Quotientenbildung die Konzentration zu bestimmen.

In einer ersten Ausführungsform der Vorrichtung durchströmt das zu untersuchende Gas seriell zunächst die Messküvette, dann die Absorptionsvorrichtung und schließlich die Referenzküvette. Ein solcher bezüglich der Gasführung serieller Aufbau ist relativ einfach und entsprechend kostenbewusst.

Um das zeitliche Ansprechverhalten auf der Mess- und der Referenzstrecke möglichst gut anzugleichen, ist in einer zweiten Ausführungsform das zu untersuchende Gas parallel durch Mess- und Referenzstrecke geführt. Dabei ist das Gas einerseits der Messküvette zugeführt und andererseits über die Absorptionsvorrichtung der Referenzküvette zugeführt. Um jetzt aber das zeitliche Ansprechverhalten anzugleichen, ist in der Zuführung zur Messküvette zusätzlich eine konstruktiv der Absorptionsvorrichtung identisch ausgebildete Strömungsangleichvorrichtung angeordnet, die jedoch keine die Messkomponente absorbierende Substanz enthält. Damit liegen sowohl im Mess- als auch im Referenzkanal gleiche Strömungsverhältnisse vor, die sich positiv auf das zeitliche Ansprechverhalten auswirken.

In Weiterbildung der Erfindung ist die Absorptionsvorrichtung als eine von dem Gas durchströmte Patrone ausgebildet, in der ein Absorber, der die absorbierende Substanz enthält, angeordnet ist. Vorteilhafterweise ist die Beladung des Absorbers mit $H_2S$ optisch, insbesondere farblich, erkennbar, so dass unmittelbar ersichtlich ist, wann der Absorber auszutauschen ist.

Als besonders selektiv $H_2S$ absorbierende Substanzen haben sich Elemente der fünften Hauptgruppe herausgestellt.

Diese liegen zur Vermeidung von $SO_2$-Absorption bevorzugt in Verbindungen mit Halogeniden vor, z.B. $SbCl_3$, $SbBr_3$, $SbCl_5$.

In Weiterbildung enthält die absorbierende Substanz ein zweikomponentiges Puffersystem, mit einer Säure mit $0 < pKs < 2$ als erste Komponente und einem Hydroxid-Salz als zweite Komponente. Eine Puffersäure mit diesem pKs-Wert vermeidet einerseits eine Reaktion mit $SO_2$ und andererseits bewirkt sie, dass die Reaktion mit $H_2S$ vollständig abläuft. Als Puffersäure kommt beispielsweise Trichloressigsäure oder Dichloressigsäure in Betracht und als Hydroxid-Salz beispielsweise NaOH, KOH, $Mg(OH)_2$, $Al(OH)_3$.

Um die Austrocknung des Absorbers zu verlangsamen, enthält die absorbierende Substanz eine hygroskopische Halogenidverbindung, z.B. $MgCl_2$ oder $AlCl_3$.

Diese wässrige Absorberlösung wird in einem geeigneten Trägermaterial, vorzugsweise Kieselgel gespeichert. Durch die Speicherung auf dem festen Material gelingt es, einen festen Absorber zu realisieren, der sich vorteilhaft in Form einer Absorptionspatrone aufbauen lässt.

Ein solcher Absorber kann bei Normaltemperaturen von ca. 0° bis ca. 100° betrieben werden.

Im Folgenden wird die Erfindung anhand eines Ausführungsbeispiels unter Bezugnahme auf die Zeichnung im Einzelnen erläutert. In der Zeichnung zeigen:

Fig. 1　einen schematischen Aufbau einer erfindungsgemäßen Vorrichtung;

Fig. 2　eine weitere Ausführungsform der Vorrichtung mit paralleler Gasführung;

Fig. 3　eine schematische Darstellung einer Absorptionsvorrichtung;

Fig. 4　eine schematische Darstellung der empfangenen Lichtintensität.

Eine erfindungsgemäße Vorrichtung 10 ist nach Art eines Fotometers aufgebaut und weist eine Lichtquelle 12, insbesondere eine UV-Lichtquelle, auf. Diese Lichtquelle 12 emittiert Licht unterschiedlicher Wellenlängen, weshalb eine Wellenlängenselektionseinheit 13 zur Auswahl einer bestimmten gewünschten Wellenlänge vorgesehen ist. Die Wellenlängenselektionseinheit 13 umfasst optische Filter 14, 15, die über ein Filterrad 16 in den Strahlengang einschwenkbar sind. Das Filterrad 16 wird angetrieben über einen Schrittmotor 18.

Das ausgesendete Licht, das in der Zeichnung mit dem Bezugszeichen 20 gekennzeichnet ist, wird durch eine Optik 22 gebündelt und trifft auf einen halbdurchlässigen Teilerspiegel 24.

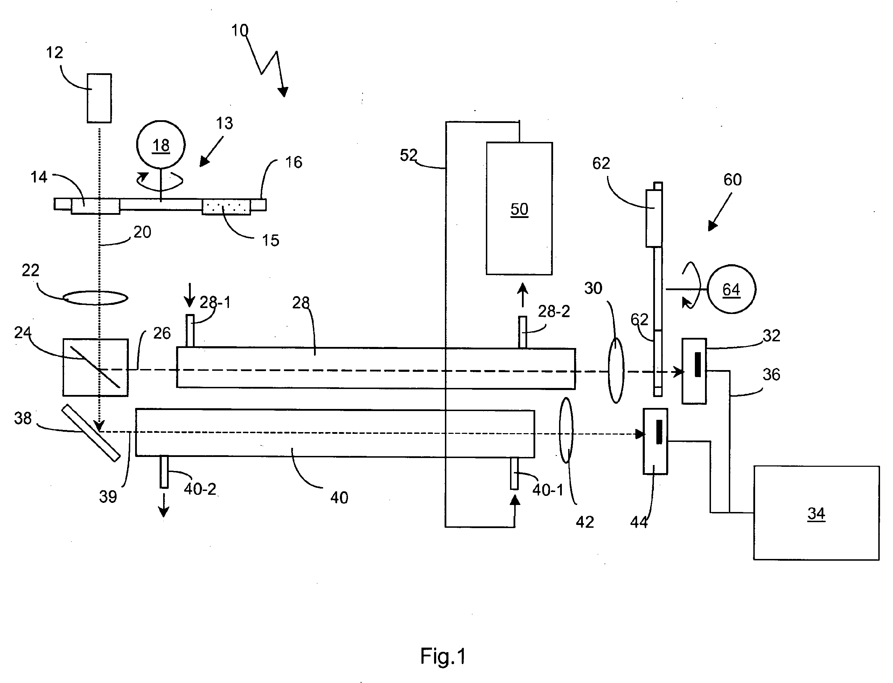

Ein erster Teil des Lichtstrahls 20, der in der Fig. 1 gestrichelt dargestellt ist und das Bezugszeichen 26 trägt, durchläuft eine Messküvette 28 und wird nach Durchlauf durch die Messküvette 28 über eine Optik 30 auf einen Messdetektor 32 geführt. Der Detektor 32 wandelt das empfangene Licht entsprechend der Intensität in ein elektrisches Signal um, das an eine Auswerteeinheit 34 über eine Leitung 36 gegeben wird.

Ein zweiter Teil 39 des Lichtstrahls 20, der durch den Teilerspiegel 24 aufgeteilt wurde, wird über einen Vollspiegel 38 durch eine Referenzküvette 40 geführt und schließlich über eine Empfangsoptik 42 auf einen Referenzdetektor 44, der, wie schon der Messdetektor 32, das Licht in elektrische Signale umwandelt und die Signale der Auswerteeinheit 34 zuführt.

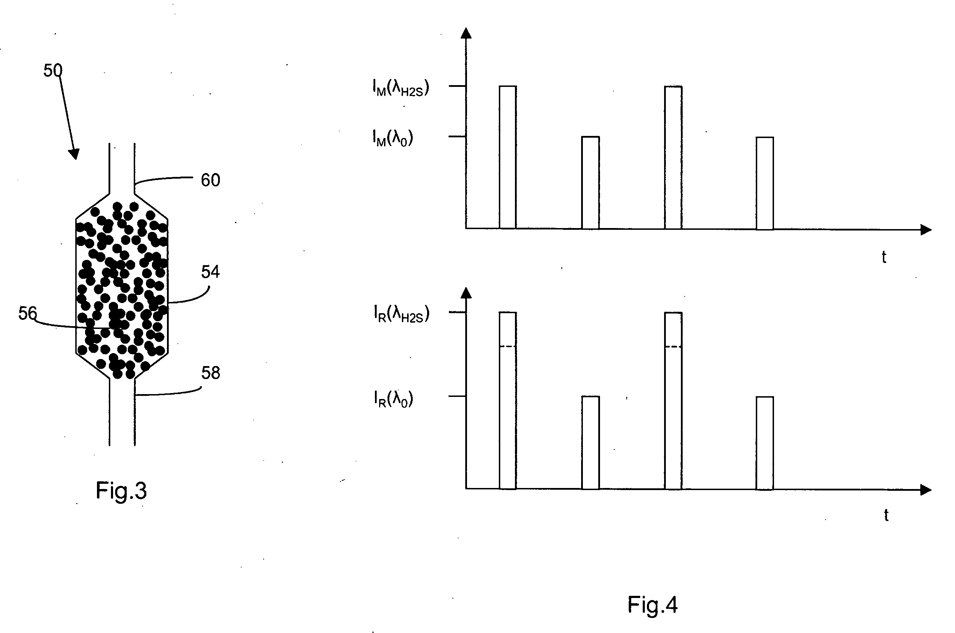

In einer ersten Ausführungsform (Fig. 1) wird das Messgas, das untersucht werden soll, also dessen Gehalt an einer gewünschten Messkomponente bestimmt werden soll, einem Messküvetteneinlass 28-1 zugeführt, von dem aus es die Messküvette 28 durchströmt und über einen Messküvettenauslass 28-2 wieder abgeführt wird. Von dem Messküvettenauslass 28-2 wird das Gas einer Absorptionsvorrichtung 50 zugeführt, in der ein Absorber 56 mit einer absorbierenden Substanz vorgesehen ist, die die Messkomponente vollständig absorbiert. Die Absorptionsvorrichtung 50 mit dem Absorber 56 wird weiter unten unter Bezugnahme auf Fig. 3 näher erläutert. Von der Absorptionsvorrichtung 50 wird das Gas, das jetzt die Messkomponente nicht mehr enthält, über eine Leitung 52 einem Referenzküvetteneinlass 40-1 zugeführt und durchläuft dann die Referenzküvette 40 bis zu einem Referenzküvettenauslass 40-2, über den das Gas schließlich aus der Referenzküvette 40 austritt. Das Gas wird in dieser Ausführungsform also seriell durch die Messküvette 28, die Absorptionsvorrichtung 50 und die Referenzküvette 40 geführt.

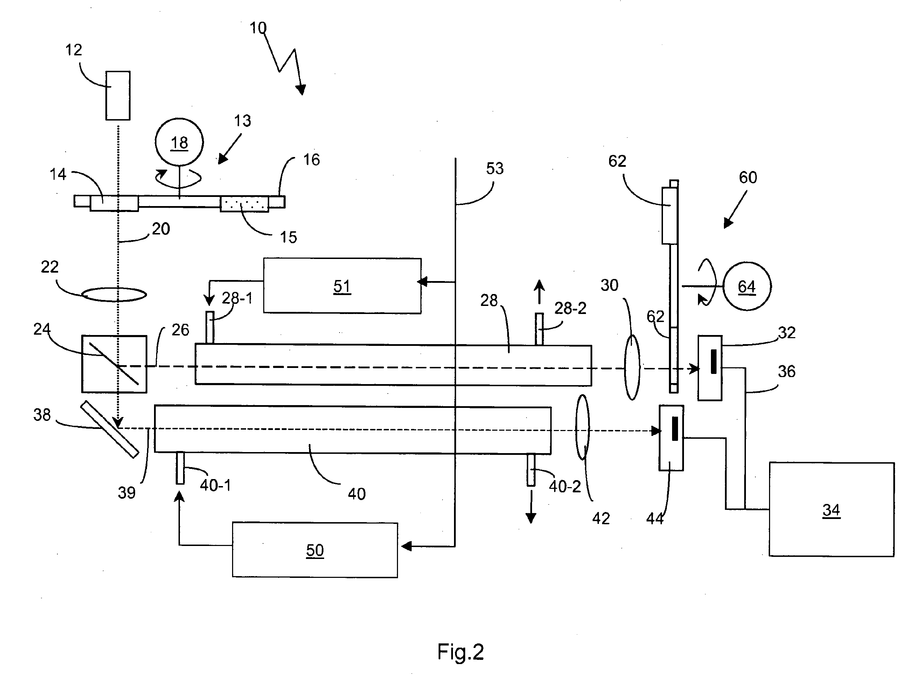

In einer zweiten Ausführungsform (Fig. 2) wird das Gas parallel durch Messküvette 28 und Referenzküvette 40 geführt. Dazu wird das Gas über eine Leitung 53 parallel der Absorptionsvorrichtung 50 und einer Strömungsangleichvorrichtung 51 zugeführt. Von der Strömungsangleichvorrichtung 51 wird das Gas dann der Messküvette 28, also dem Messküvetteneinlass 28-1, zugeführt. Im anderen Zweig wird das Gas von der Absorptionsvorrichtung 50 der Referenzküvette, also dem Referenzküvetteneinlass 40-1, zugeführt. Die beiden Parallelzweige bestehen also einerseits aus der Strömungsangleichvorrichtung 51 und der Messküvette 28 (Messzweig) und andererseits aus der Absorptionsvorrichtung 50 und der Referenzküvette 40 (Referenzzweig). Dabei ist die Strömungsangleichvorrichtung 51 konstruktiv identisch zur Absorptionsvorrichtung 50 aufgebaut, allerdings enthält die Strömungsangleichvorrichtung 51 keine absorbierende Substanz. Sie dient damit nur zur Angleichung der Gasströmungsverhältnisse.

Die Vorrichtung 10 ist dafür geeignet, $H_2S$ als Messkomponente eines Gases zu messen. Das bedeutet, dass in der Absorptionsvorrichtung 50 $H_2S$ vollständig absorbiert werden soll. Dazu ist die Absorptionsvorrichtung 50 wie folgt aufgebaut (Fig. 3):

Die Absorptionsvorrichtung 50 weist einen patronenartigen Körper 54 auf, in dem die eigentlich absorbierende Substanz angeordnet ist. In Fig. 3 ist mit der Bezugsziffer 56 der Absorber, bestehend aus Trägermaterial und absorbierender Substanz, bezeichnet. Die Patrone 54 ist so ausgebildet, dass das Gas, das auf der einen Stirnseite 58 eintritt und auf der anderen Stirnseite 60 wieder austritt, möglichst den gesamten Absorber 56 durchfließt.

Ausgangspunkt der absorbierenden Substanz sind Stoffe der fünften Hauptgruppe, die $H_2S$ selektiv absorbieren, wie z.B. As, Sb, Bi. Um $SO_2$-Absorptionen zu vermeiden, werden Verbindungen dieser Stoffe mit Halogeniden gewählt, wie z.B. $SbCl_3$, $SbBr_3$ oder $SbCl_5$. Um eine Reaktion des Absorbers mit $SO_2$ zu vermeiden, muss eine Puffersäure mit einem pKs-Wert < 2 gewählt werden, und um die $H_2S$-Aktion vollständig ablaufen zu lassen, sollte pKs > 0 sein. Damit stellt sich ein pH-Wert zwischen 0 und 2 ein. Das Puffersystem selbst besteht nun aus einer ersten Komponente, nämlich einer Säure mit 0 < pKs < 2, beispielsweise Trichloressigsäure oder Dichloressigsäure und einer zweiten Komponente in Form eines Hydroxidsalzes, wie z.B. NaOH, KOH, $Mg(OH)_2$ oder $Al(OH)_3$. Durch eine Teilneutralisation der ersten Komponente mit der zweiten Komponente entsteht ein Puffersystem mit einem pH-Wert zwischen 0 und 2. Weiterhin werden dem Absorber eine oder mehrere stark hygroskopische Halogenidverbindungen hinzugefügt, um die Austrocknung des Absorbers zu verlangsamen. Eine solche Halogenidverbindung kann z.B. $MgCl_2$ oder $AlCl_3$ oder dergleichen sein. Die so angesetzte wässrige Absorberlösung wird in einem geeigneten Trägermaterial gespeichert. In vorteilhafter Weise ist dieses Trägermaterial ein Kieselgel mit einer Korngröße von mehreren Millimetern. Durch die Speicherung der wässrigen Absorberlösung in dem Kieselgel gelingt es, einen festen Absorber 56 zu realisieren, der sich in Form der Patrone 54 gut aufbauen lässt. Dieser Absorber 56 hat auch die Eigenschaft, dass er sich bei Beladung mit $H_2S$ verfärbt, so dass man an der Verfärbung des Inhalts der Patrone 54 feststellen kann, wann die absorbierende Substanz vollständig verbraucht ist. Dann hat sich nämlich der gesamte Absorber 56 von dem Absorbereingang 58 ausgehend bis hin zum Auslass 60 verfärbt.

Im Folgenden wird nun erläutert, wie die Konzentration der Messkomponente bestimmt wird.

In einer ersten sehr einfachen Ausführungsform des Verfahrens wird das Grundprinzip deutlich. Zunächst wird einer der Filter 14 in den Lichtstrahl gebracht, so dass der Lichtstrahl 20 eine gewünschte Wellenlänge enthält, bei der die Messkomponente ($H_2S$) eine Absorption zeigt. Diese Wellenlänge soll im Folgenden Absorptionswellenlänge genannt werden.

Der erste Teil 26 des Lichtstrahls 20 durchläuft die Messküvette 28, in der das Licht zumindest teilweise absorbiert wird, und zwar von den Komponenten des Messgases und insbesondere auch von der Messkomponente. Der so geschwächte Lichtstrahl 26 trifft dann auf den Detektor 32 und wird dort registriert mit einer Intensität $I_M(\lambda_{Abs})$. $I_M(\lambda_{Abs})$ beschreibt also die Intensität am Messdetektor bei der Absorptionswellenlänge $\lambda_{Abs}$.

Im parallelen Zweig durchläuft der zweite Teil 39 des Lichts die Referenzküvette 40. Da die Referenzküvette dasselbe Gas enthält, allerdings vermindert um die Messkomponente, treten dort dieselben Absorptionen auf mit Ausnahme der Absorption durch die Messkomponente. Deshalb wird am Referenzdetektor 44 bei der Absorptionswellenlänge eine höhere Intensität $I_R(\lambda_{Abs})$ gemessen.

Die Differenz zwischen diesen beiden Intensitäten $I_M(\lambda_{Abs})$ - $I_R(\lambda_{Abs})$ entspricht damit der Absorption durch die Messkomponente in der Messküvette 28, so dass die Auswerteeinheit 34 daraus die Konzentration der Messkomponente bestimmen kann.

Dieser Zusammenhang ist auch in Fig. 4 angedeutet. Die Intensitätsveränderung, die der Absorption durch die Messkomponente entspricht, ist dargestellt als der Anteil von $I_R(\lambda_{Abs})$, der oberhalb der gestrichelten Linie liegt.

Um aber eine gute Langzeitstabilität zu erhalten und möglichst unabhängig zu sein von etwaigen Driften, beispielsweise aufgrund von Temperaturen oder Alterungserscheinungen, ist in einer weiteren Ausführungsform des erfindungsgemäßen Verfahrens vorgesehen, die Intensitätsmessungen nicht nur bei der Absorptionswellenlänge vorzunehmen, sondern auch bei einer Nichtabsorptionswellenlänge $\lambda_0$, also bei einer Wellenlänge, bei der die Messkomponente nicht absorbiert. Die Nichtabsorptionswellenlänge $\lambda_0$ wird dadurch erzeugt, dass ein entsprechendes Filter 15 des Filterrades 16 in den Strahlengang eingeschwenkt wird. Die entsprechenden Intensitäten, die dann am Messdetektor 32 und am Referenzdetektor 44 gemessen werden, werden mit $I_M(\lambda_0)$ und $I_R(\lambda_0)$ (siehe auch Fig. 4) bezeichnet und werden genauso bestimmt wie die Intensitäten bei der Absorptionswellenlänge. Dann kann die Konzentration der Messkomponente in an sich bekannter Weise aus der doppelten Quotientenbildung bestimmt werden, so dass dabei Änderungen aufgrund von Driften, Alterungserscheinungen und dgl. größtenteils kompensiert werden.

$$\frac{\dfrac{I_M(\lambda_0)}{I_R(\lambda_0)} - \dfrac{I_M(\lambda_{Abs})}{I_R(\lambda_{Abs})}}{\dfrac{I_M(\lambda_0)}{I_R(\lambda_0)}}.$$

Zu Kalibrier- und Justagezwecken kann eine Justiereinheit 60 vorgesehen sein, die die Prüfküvette 62 umfasst, die zu Kalibrier- und Justierzwecken in den Strahlengang mittels eines Motors 64 eingeschwenkt werden kann, so dass eine Kalibrierung auf bekannte Größen vorgenommen werden kann.

**Patentansprüche**

1. Vorrichtung zur Bestimmung einer Konzentration einer Messkomponente in einem Gas, mit

   - einer Lichtquelle (12),
   - einer Messküvette (28),
   - einer im optischen Strahlengang parallel dazu angeordneten Referenzküvette (40),
   - wenigstens einem Lichtempfänger (32, 44)
   - und einer Auswerteeinheit (34), die aus den Signalen des Lichtempfängers (32, 44) die Konzentration bestimmt,

   wobei das zu untersuchende Gas einerseits der Messküvette (28) und andererseits über eine Absorptionsvorrichtung (50), die eine die Messkomponente vollständig absorbierende Substanz enthält, der Referenzküvette (40) zugeführt ist wobei die Messkomponente $H_2S$ ist und wobei eine Wellenlängenselektionseinheit (13) vorgesehen ist zur Auswahl einer Absorptionswellenlänge ($\lambda_{Abs}$).

2. Vorrichtung nach Anspruch 1, **dadurch gekennzeichnet, dass** das zu untersuchende Gas seriell zunächst die Messküvette (28) durchströmt, dann durch die Absorptionsvorrichtung (50) und schließlich durch die Referenzküvette (40) geführt ist.

3. Vorrichtung nach Anspruch 1, **dadurch gekennzeichnet, dass** das zu untersuchende Gas parallel einerseits der Messküvette (28) zugeführt ist und andererseits über die Absorptionsvorrichtung (50) in die Referenzküvette (40) geführt ist, wobei in der Zuführung zur Messküvette (28) eine Strömungsangleichvorrichtung (51) angeordnet ist, die konstruktiv zu der ersten Absorptionsvorrichtung (50) identisch ausgebildet ist, aber keine die Messkomponente absorbierende Substanz enthält.

4. Vorrichtung nach einem der vorhergehenden Ansprüche, **dadurch gekennzeichnet, dass** die Absorptionsvorrichtung (50) als eine von dem Gas durchströmte Patrone (54) ausgebildet ist, in der ein Absorber (56), der die absorbierende Substanz enthält, angeordnet ist.

5. Vorrichtung nach Anspruch 4, **dadurch gekennzeichnet, dass** der Absorber (56) Kieselgel als Träger für die absorbierende Substanz enthält.

6. Vorrichtung nach Anspruch 5, **dadurch gekennzeichnet, dass** die absorbierende Substanz Elemente der fünften Hauptgruppe umfasst, die insbesondere in Verbindungen mit Halogeniden vorliegen.

7. Vorrichtung nach Anspruch 6, **dadurch gekennzeichnet, dass** die absorbierende Substanz ein zweikomponentiges Puffersystem enthält, mit einer Säure mit 0 < pKs < 2 als erste Komponente und einem Hydroxid-Salz als zweite Komponente.

8. Vorrichtung nach einem der vorhergehenden Ansprüche 6 oder 7, **dadurch gekennzeichnet, dass** die absorbierende Substanz eine hygroskopische Halogenidverbindung enthält..

9. Vorrichtung nach einem der vorhergehenden Ansprüche 4 bis 9, **dadurch gekennzeichnet, dass** die Beladung des Absorbers (56) mit $H_2S$ optisch, insbesondere farblich, erkennbar ist.

10. Vorrichtung nach einem der vorhergehenden Ansprüche, **dadurch gekennzeichnet, dass** sowohl für das die Messküvette (28) durchlaufende Licht (26) als auch für das die Referenzküvette (40) durchlaufende Licht (39) jeweils ein Lichtempfänger (32 bzw. 44) vorgesehen ist und die Auswerteeinheit (34) dazu ausgebildet ist, mittels der doppelten Quotientenbildung die Konzentration zu bestimmen.

**Claims**

1. An apparatus for the determination of a concentration of a component to be measured in a gas, comprising:

   - a light source (12);
   - a measurement cuvette (28);
   - a reference cuvette (40) arranged in the optical beam path in parallel to the measurement cuvette;
   - at least one light receiver (32, 44);
   - and an evaluation unit (34) which determines the concentration from the signals of the light receiver (32, 44),

wherein the gas to be analyzed is supplied to the measurement cuvette (28), on the one hand, and, on the other hand, via an absorption apparatus (50) which includes a substance which completely absorbs the component to be measured to the reference cuvette (40), wherein the component to be measured is $H_2S$ and wherein a wavelength selection unit (13) is provided for the selection of an absorption wavelength ($\lambda_{Abs}$).

2. An apparatus in accordance with claim 1, wherein the gas to be analyzed flows serially, initially through the measurement cuvette (28), then through the absorption apparatus (50) and is finally guided through the reference cuvette (40).

3. An apparatus in accordance with claim 1, wherein the gas to be analyzed is supplied in parallel to the measurement cuvette (38), on the one hand, and, on the other hand, via the absorption apparatus (50) into the reference cuvette (40), wherein a current matching apparatus (51) is arranged in the supply for the measurement cuvette (28) which, from a construction point of view, is of identical design to the first absorption apparatus (50), but does not include any substance absorbing the component to be measured.

4. An apparatus in accordance with any preceding claim, wherein the absorption apparatus (50) is configured as a cartridge (54) flowed through by the gas, in which an absorber (56) is arranged which includes the absorbing substance.

5. An apparatus in accordance with claim 4, wherein the absorber (56) includes silica gel as a carrier for the absorbing substance.

6. An apparatus in accordance with claim 5, wherein the absorbing substance comprises elements of the fifth main group, which preferably are present in combination with halides.

7. An apparatus in accordance with claim 6, wherein the absorbing substance includes a two-component buffer system having an acid with $0 < pKs < 2$ as a first component and a hydroxide salt as a second component.

8. An apparatus in accordance with claim 6 or 7, wherein the absorbing substance includes a hygroscopic halide compound.

9. An apparatus in accordance with any preceeding claim 4 to 9, wherein the loading of the absorber (56) with $H_2S$ is optically detectable, especially in terms of color.

10. An apparatus in accordance with claim 1, wherein a respective light receiver (32 or 34 respectively) is provided, both for the light (26) passing through the measurement cuvette (28) and for the light (39) passing through the reference cuvette (40) and the evaluation unit (34) is configured to determine the concentration by means of the double formation of quotients.

**Revendications**

1. Appareil pour la détermination d'une concentration d'un composant à être mesuré dans un gaz, comprenant:

   - une source de lumière (12);
   - une cuvette de mesure (28);
   - une cuvette de référence (40) disposé dans le trajet de faisceau optique en parallèle à la cuvette de mesure;
   - au moins un récepteur de lumière (32, 44);
   - et une unité d'évaluation (34) qui détermine la concentration à partir des signaux du récepteur de lumière (32, 44),

   dans lequel le gaz à analyser est amené à la cuvette de mesure (28), d'une part, et, d'autre part, par l'intermédiaire d' un dispositif d' absorption (50) qui comprend une substance qui absorbe complètement le composant à mesurer à la référence cuvette (40), dans lequel le composant à mesurer est H2S et dans lequel une unité de sélection de longueur d'onde (13) est prévu pour la sélection d' une longueur d'onde d'absorption ($\lambda_{Abs}$).

2. Appareil selon la revendication 1, dans lequel le gaz à analyser s'écoule en série, d'abord à travers la cuvette de mesure (28), puis à travers le dispositif d'absorption (50) et est enfin guidé à travers la cuvette de référence (40).

3. Appareil selon la revendication 1, dans lequel le gaz à analyser est alimenté en parallèle à la cuvette de mesure (38), d'une part, et, d'autre part, par l'intermédiaire de l'dispositif d' absorption (50) dans la cuvette de référence (40), dans lequel un dispositif d'adaptation de courant (51) est disposé dans l'alimentation de la cuvette de mesure (28), qui, d'un point de vue de la construction, est de conception identique au premier appareil d'absorption (50), mais ne comprend pas tout en absorbant la composante à mesurer substance.

4. Appareil selon l'une quelconque des revendications précédentes, dans lequel l'absorption appareil (50) est configuré comme une cartouche (54) traversé par le gaz, dans lequel un absorbeur (56) est disposé, qui comprend la substance absorbante.

5.  Appareil selon la revendication 4, dans lequel l'absorbeur (56) comprend un gel de silice comme support pour la substance absorbante.

6.  Appareil selon la revendication 5, dans lequel la substance comprend des éléments absorbants du cinquième groupe principal, qui sont de préférence présents en combinaison avec des halogénures.

7.  Appareil selon la revendication 6, dans lequel la substance absorbant comprend un système tampon à deux composants ayant un acide avec $0 < pK < 2$ en tant que premier composant et un sel d'hydroxyde, comme second composant.

8.  Appareil selon les revendications 6 ou 7, dans lequel la substance absorbante comprend un composé d'halogénure hygroscopique.

9.  Appareil selon l'une quelconque des revendications précédentes 4 à 9, dans lequel le chargement de l'absorbeur (56) avec $H_2S$ est détectable optiquement, en particulier en termes de couleur.

10. Appareil selon la revendication 1, dans lequel un récepteur de lumière respectif (32 ou 34 respectivement) est prévue, à la fois pour la lumière (26) passant à travers la cuvette de mesure (28) et de la lumière (39) passant à travers la cuvette de référence (40) et l'unité d'évaluation (34) est configuré pour déterminer la concentration à l'aide de la double formation de quotients.

Fig.1

Fig.2

Fig.3

Fig.4

$I_M(\lambda_{H2S})$

$I_M(\lambda_0)$

$I_R(\lambda_{H2S})$

$I_R(\lambda_0)$

50
60
54
56
58

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3173016 A

- DE 10242355